# EUROPEAN PATENT APPLICATION

(11) **EP 4 592 316 A1**
(43) Date of publication of application: **30.07.2025**
(21) Application number: 23867637.3
(22) Date of filing: 22.09.2023
(51) Int. Cl.: C07K 16/28, A61K 39/395, A61P 35/00

(54) **ISOLATED ANTIGEN-BINDING PROTEIN AND USE THEREOF**

(30) Priority: 23.09.2022 CN 202211162830
(71) Applicant: Lindis Biotech GmbH, 82178 Puchheim (DE)
(72) Inventor: Horst Lindhofer, 82178 Puchheim (DE); SUN, Zhe, Guangzhou, Guangdong 510110 (CN); LI, Dongchen, Guangzhou, Guangdong 510110 (CN)
(74) Representative: Isarpatent
(86) International application number: PCT/CN2023/120746
(87) International publication number: WO 2024/061352

(57) **Abstract**

The present invention relates to an isolated antigen-binding protein, which comprises a CD3-binding moiety, wherein the CD3-binding moiety comprises amino acid sequences having at least 95% identity to heavy chain variable regions HCDR1, HCDR2 and HCDR3; the amino acid sequence of the HCDR1 is set forth in any one of SEQ ID NOs: 1 and 2; the amino acid sequence of the HCDR2 is set forth in any one of SEQ ID NOs: 3, 4 and 5; the amino acid sequence of the HCDR3 is set forth in SEQ ID NO: 6; and/or, the CD3-binding moiety comprises amino acid sequences having at least 95% identity to light chain variable regions LCDR1, LCDR2 and LCDR3; the amino acid sequence of the LCDR1 is set forth in any one of SEQ ID NOs: 7, 8 and 9; the amino acid sequence of the LCDR2 is set forth in any one of SEQ ID NOs: 11 and 12; the amino acid sequence of the LCDR3 is set forth in SEQ ID NO: 13, wherein the CD3-binding moiety can induce T cell activation.

## Description

This application claims the priority of Chinese Patent Application No. CN202211162830.5, filed with the China National Intellectual Property Administration on September 23, 2022, the contents described in the description, the drawings and the claims of the priority document are incorporated in the description of the present disclosure by reference in their entirety as part of the original description of the present disclosure. The applicant further states that the applicant has the right to amend the description and claims of the present disclosure based on the priority document.

### FIELD

The present disclosure relates to the field of biomedicine, in particular to an antigen-binding protein comprising a CD3-binding moiety.

### BACKGROUND

Currently, bispecific antibodies and antibody fragments have been explored as means to recruit cytolytic T cells and to kill tumor cells. However, the clinical use of many T cell-recruiting bispecific antibodies is limited by challenges including excessive cytokine release, potential immunogenicity and manufacturing issues. Therefore, there is a great need for developing a tumor-targeting bispecific antibody that can recruit catalytic T-cells to kill tumor cells, and has reduced toxicity and improved manufacturability.

The human T cell antigen receptor protein-CD3 complex is composed of six distinct chains: a CD3γ chain (SwissProt P09693), a CD3δ chain (SwissProt P04234), two CD3ε chains (SwissProt P07766), and a homodimer of CD3ζ chains (SwissProt P20963) (ε γ: ε δ: ζ ζ). The homodimer is associated with the α and β chains of T cell receptor. The complex plays an important role in coupling antigen recognition to several intracellular signal-transduction pathways. The CD3 complex mediates signal transduction, resulting in T cell activation and proliferation. CD3 is necessary for the immune response.

### SUMMARY

The present application provides an isolated antigen-binding protein comprising CD3-binding moiety, wherein the CD3-binding moiety comprises an amino acid sequence having at least 95% identity to HCDR1, HCDR2 and HCDR3 of heavy chain variable region, wherein the HCDR1 has an amino acid sequence set forth in any one of SEQ ID NOs: 1 and 2, the HCDR2 has an amino acid sequence set forth in any one of SEQ ID NOs: 3, 4 and 5, and the HCDR3 has an amino acid sequence set forth in SEQ ID NO: 6; and/or
the CD3-binding moiety comprises an amino acid sequence having at least 95% identity to LCDR1, LCDR2 and LCDR3 of light chain variable region, wherein the LCDR1 has an amino acid sequence set forth in any one of SEQ ID NOs: 7, 8 and 9, the LCDR2 has an amino acid sequence set forth in any one of SEQ ID NOs: 11 and 12, and the LCDR3 has an amino acid sequence set forth in SEQ ID NO: 13; and wherein the CD3-binding moiety is capable of inducing activation of T cells.

In some embodiments, the antigen-binding protein comprises a CD3-binding moiety, wherein the CD3-binding moiety comprises HCDR1, HCDR2 and HCDR3 of heavy chain variable region, wherein the HCDR1 has an amino acid sequence set forth in any one of SEQ ID NOs: 1 and 2, the HCDR2 has an amino acid sequence set forth in any one of SEQ ID NOs: 3, 4 and 5, and the HCDR3 has an amino acid sequence set forth in SEQ ID NO: 6; and/or
the CD3-binding moiety comprises LCDR1, LCDR2 and LCDR3 of light chain variable region, wherein the LCDR1 has an amino acid sequence set forth in any one of SEQ ID NOs: 7, 8 and 9, the LCDR2 has an amino acid sequence set forth in any one of SEQ ID NOs: 11 and 12, and the LCDR3 has an amino acid sequence set forth in SEQ ID NO: 13; and wherein the CD3-binding moiety is capable of inducing activation of T cells.

In some embodiments, the antigen-binding protein comprises a CD3-binding moiety, wherein the CD3-binding moiety comprises HCDR1, HCDR2 and HCDR3 of heavy chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6,
the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6,
the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6,
the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6,
the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6, or
the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6.

In some embodiments, the antigen-binding protein comprises a CD3-binding moiety, wherein the CD3-binding moiety comprises LCDR1, LCDR2 and LCDR3 of light chain variable region, wherein the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13,
the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13,
the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13,
the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13,
the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13, or
the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13.

For example, in some embodiments, the antigen-binding protein comprises a CD3-binding moiety, wherein the CD3-binding moiety comprises HCDR1, HCDR2 and HCDR3 of heavy chain variable region, and comprises LCDR1, LCDR2 and LCDR3 of light chain variable region, wherein the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6; and/or the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

In some embodiments, the antigen-binding protein comprises a CD3-binding moiety, wherein the CD3-binding moiety comprises an amino acid sequence having at least 80% identity to a heavy chain variable region having an amino acid sequence set forth in any one of SEQ ID NOs: 14 to 18; and/or the CD3-binding moiety comprises an amino acid sequence having at least 80% identity to a light chain variable region having an amino acid sequence set forth in any one of SEQ ID NOs: 19 to 23.

In some embodiments, the antigen-binding protein comprises a CD3-binding moiety, wherein the CD3-binding moiety comprises a heavy chain variable region having an amino acid sequence set forth in any one of SEQ ID NOs: 14 to 18; and/or the CD3-binding moiety comprises a light chain variable region having an amino acid sequence set forth in any one of SEQ ID NOs: 19 to 23.

In some embodiments, the antigen-binding protein comprises a CD3-binding moiety, wherein the CD3-binding moiety comprises:
a heavy chain variable region having an amino acid sequence set forth in SEQ ID NO: 15 and a light chain variable region having an amino acid sequence set forth in SEQ ID NO: 19,
a heavy chain variable region having an amino acid sequence set forth in SEQ ID NO: 16 and a light chain variable region having an amino acid sequence set forth in SEQ ID NO: 19,
a heavy chain variable region having an amino acid sequence set forth in SEQ ID NO: 17 and a light chain variable region having an amino acid sequence set forth in SEQ ID NO: 19,
a heavy chain variable region having an amino acid sequence set forth in SEQ ID NO: 18 and a light chain variable region having an amino acid sequence set forth in SEQ ID NO: 19,
a heavy chain variable region having an amino acid sequence set forth in SEQ ID NO: 14 and a light chain variable region having an amino acid sequence set forth in SEQ ID NO: 20,
a heavy chain variable region having an amino acid sequence set forth in SEQ ID NO: 15 and a light chain variable region having an amino acid sequence set forth in SEQ ID NO: 20,
a heavy chain variable region having an amino acid sequence set forth in SEQ ID NO: 16 and a light chain variable region having an amino acid sequence set forth in SEQ ID NO: 20,
a heavy chain variable region having an amino acid sequence set forth in SEQ ID NO: 17 and a light chain variable region having an amino acid sequence set forth in SEQ ID NO: 20,
a heavy chain variable region having an amino acid sequence set forth in SEQ ID NO: 18 and a light chain variable region having an amino acid sequence set forth in SEQ ID NO: 20,
a heavy chain variable region having an amino acid sequence set forth in SEQ ID NO: 14 and a light chain variable region having an amino acid sequence set forth in SEQ ID NO: 21,
a heavy chain variable region having an amino acid sequence set forth in SEQ ID NO: 15 and a light chain variable region having an amino acid sequence set forth in SEQ ID NO: 21,
a heavy chain variable region having an amino acid sequence set forth in SEQ ID NO: 16 and a light chain variable region having an amino acid sequence set forth in SEQ ID NO: 21,
a heavy chain variable region having an amino acid sequence set forth in SEQ ID NO: 17 and a light chain variable region having an amino acid sequence set forth in SEQ ID NO: 21,
a heavy chain variable region having an amino acid sequence set forth in SEQ ID NO: 18 and a light chain variable region having an amino acid sequence set forth in SEQ ID NO: 21,
a heavy chain variable region having an amino acid sequence set forth in SEQ ID NO: 14 and a light chain variable region having an amino acid sequence set forth in SEQ ID NO: 22,
a heavy chain variable region having an amino acid sequence set forth in SEQ ID NO: 15 and a light chain variable region having an amino acid sequence set forth in SEQ ID NO: 22,
a heavy chain variable region having an amino acid sequence set forth in SEQ ID NO: 16 and a light chain variable region having an amino acid sequence set forth in SEQ ID NO: 22,
a heavy chain variable region having an amino acid sequence set forth in SEQ ID NO: 17 and a light chain variable region having an amino acid sequence set forth in SEQ ID NO: 22,
a heavy chain variable region having an amino acid sequence set forth in SEQ ID NO: 18 and a light chain variable region having an amino acid sequence set forth in SEQ ID NO: 22,
a heavy chain variable region having an amino acid sequence set forth in SEQ ID NO: 14 and a light chain variable region having an amino acid sequence set forth in SEQ ID NO: 23,
a heavy chain variable region having an amino acid sequence set forth in SEQ ID NO: 15 and a light chain variable region having an amino acid sequence set forth in SEQ ID NO: 23,
a heavy chain variable region having an amino acid sequence set forth in SEQ ID NO: 16 and a light chain variable region having an amino acid sequence set forth in SEQ ID NO: 23,
a heavy chain variable region having an amino acid sequence set forth in SEQ ID NO: 17 and a light chain variable region having an amino acid sequence set forth in SEQ ID NO: 23,
a heavy chain variable region having an amino acid sequence set forth in SEQ ID NO: 18 and a light chain variable region having an amino acid sequence set forth in SEQ ID NO: 23,
a heavy chain variable region having an amino acid sequence set forth in SEQ ID NO: 37 and a light chain variable region having an amino acid sequence set forth in SEQ ID NO: 40,
a heavy chain variable region having an amino acid sequence set forth in SEQ ID NO: 37 and a light chain variable region having an amino acid sequence set forth in SEQ ID NO: 39,
a heavy chain variable region having an amino acid sequence set forth in SEQ ID NO: 37 and a light chain variable region having an amino acid sequence set forth in SEQ ID NO: 20,
a heavy chain variable region having an amino acid sequence set forth in SEQ ID NO: 37 and a light chain variable region having an amino acid sequence set forth in SEQ ID NO: 21,
a heavy chain variable region having an amino acid sequence set forth in SEQ ID NO: 37 and a light chain variable region having an amino acid sequence set forth in SEQ ID NO: 22,
a heavy chain variable region having an amino acid sequence set forth in SEQ ID NO: 37 and a light chain variable region having an amino acid sequence set forth in SEQ ID NO: 23,
a heavy chain variable region having an amino acid sequence set forth in SEQ ID NO: 38 and a light chain variable region having an amino acid sequence set forth in SEQ ID NO: 40,
a heavy chain variable region having an amino acid sequence set forth in SEQ ID NO: 38 and a light chain variable region having an amino acid sequence set forth in SEQ ID NO: 39,
a heavy chain variable region having an amino acid sequence set forth in SEQ ID NO: 38 and a light chain variable region having an amino acid sequence set forth in SEQ ID NO: 20,
a heavy chain variable region having an amino acid sequence set forth in SEQ ID NO: 38 and a light chain variable region having an amino acid sequence set forth in SEQ ID NO: 21,
a heavy chain variable region having an amino acid sequence set forth in SEQ ID NO: 38 and a light chain variable region having an amino acid sequence set forth in SEQ ID NO: 22,
a heavy chain variable region having an amino acid sequence set forth in SEQ ID NO: 38 and a light chain variable region having an amino acid sequence set forth in SEQ ID NO: 23,
a heavy chain variable region having an amino acid sequence set forth in SEQ ID NO: 36 and a light chain variable region having an amino acid sequence set forth in SEQ ID NO: 40,
a heavy chain variable region having an amino acid sequence set forth in SEQ ID NO: 36 and a light chain variable region having an amino acid sequence set forth in SEQ ID NO: 39,
a heavy chain variable region having an amino acid sequence set forth in SEQ ID NO: 36 and a light chain variable region having an amino acid sequence set forth in SEQ ID NO: 20,
a heavy chain variable region having an amino acid sequence set forth in SEQ ID NO: 36 and a light chain variable region having an amino acid sequence set forth in SEQ ID NO: 21,
a heavy chain variable region having an amino acid sequence set forth in SEQ ID NO: 36 and a light chain variable region having an amino acid sequence set forth in SEQ ID NO: 22,
a heavy chain variable region having an amino acid sequence set forth in SEQ ID NO: 36 and a light chain variable region having an amino acid sequence set forth in SEQ ID NO: 23,
a heavy chain variable region having an amino acid sequence set forth in SEQ ID NO: 15 and a light chain variable region having an amino acid sequence set forth in SEQ ID NO: 40,
a heavy chain variable region having an amino acid sequence set forth in SEQ ID NO: 15 and a light chain variable region having an amino acid sequence set forth in SEQ ID NO: 39,
a heavy chain variable region having an amino acid sequence set forth in SEQ ID NO: 16 and a light chain variable region having an amino acid sequence set forth in SEQ ID NO: 40,
a heavy chain variable region having an amino acid sequence set forth in SEQ ID NO: 16 and a light chain variable region having an amino acid sequence set forth in SEQ ID NO: 39,
a heavy chain variable region having an amino acid sequence set forth in SEQ ID NO: 17 and a light chain variable region having an amino acid sequence set forth in SEQ ID NO: 40,
a heavy chain variable region having an amino acid sequence set forth in SEQ ID NO: 17 and a light chain variable region having an amino acid sequence set forth in SEQ ID NO: 39,
a heavy chain variable region having an amino acid sequence set forth in SEQ ID NO: 18 and a light chain variable region having an amino acid sequence set forth in SEQ ID NO: 40, or
a heavy chain variable region having an amino acid sequence set forth in SEQ ID NO: 18 and a light chain variable region having an amino acid sequence set forth in SEQ ID NO: 39.

In some embodiments, the antigen-binding protein is an antibody or an antigen-binding fragment thereof.

In some embodiments, the antigen-binding protein comprises a CD3-binding moiety, wherein the antibody is a monoclonal antibody.

In some embodiments, the antibody is a chimeric antibody, a humanized antibody or a fully human antibody.

In some embodiments, the antigen-binding fragment includes Fab, Fab', Fv fragment, F(ab')₂, scFv, di-scFv and/or dAb.

In some embodiments, the antigen-binding protein comprises a constant region from IgA, IgG, IgD, IgE or IgM.

In some embodiments, the CD3-binding moiety comprises a heavy chain constant region of an antibody, and the heavy chain constant region of an antibody includes a constant region from human IgG.

In some embodiments, the CD3-binding moiety comprises a heavy chain constant region of an antibody, and the heavy chain constant region of an antibody includes a heavy chain constant region from human IgG1, IgG2, IgG3 or IgG4.

In some embodiments, the heavy chain constant region of an antibody comprises an amino acid sequence set forth in SEQ ID NO: 24.

In some embodiments, the CD3-binding moiety comprises a light chain constant region of an antibody, and the light chain constant region of an antibody includes a constant region of human Ig κ light chain or a constant region of human Ig λ light chain.

In some embodiments, the light chain constant region of an antibody comprises an amino acid sequence set forth in SEQ ID NO: 25.

In some embodiments, the CD3-binding moiety comprises a heavy chain of an antibody having an amino acid sequence set forth in any one of SEQ ID NOs: 26 to 30 and a light chain of an antibody having an amino acid sequence set forth in any one of SEQ ID NOs: 31 to 35.

In some embodiments, the antigen-binding protein includes a multi-specific antibody.

In some embodiments, the multi-specific antigen-binding protein is in a form selected from the group consisting of bispecific antibody, bispecific diabody, bispecific scFv, TandAb, trivalent binding molecule and tetravalent binding molecule.

In some embodiments, the multi-specific antigen-binding protein comprises a tumor-associated antigen (TAA)-binding moiety capable of binding to at least one TAA.

In some embodiments, the TAA is selected from the group consisting of EPCAM, CCR5, CD19, HER2, HER3neu, HER3, HER4, EGFR, PSMA, CEA, MUC1, MUC2, MUC3, MUC4, MUC5, MUC7, β-hCG, Lewis-Y, CD20, CD33, CD30, ganglioside GD3, 9-O-Acetyl-GD3, GM2, globo H, fucosyl GM1, Poly SA, GD2, RON, c-Met, CEACAM-6, PCTA-1, PSA, PAP, ALCAM (CD166), PECAM-1, CD151, MAGE-1, TROP2, IGF1R, TGFBR2, GHRHR, GHR, IL-6R, gp130, TNFR2, OSMRp, Patched-1, Frizzled, Robol, LTpR, CD26, CD27, CD44, CD80, CD81, CD86, CD100, CXCR4, SAS, BCMA, TWEAKR/Fn14, FGFR4, VEGFR1,VEGFR2, SSX1, SSX2, Carboanhydrase IX (MN/CAIX), CD44v6, Sonic Hedgehog (Shh), Wue-1, plasma cell antigen, (membrane-bound) IgE, melanoma chondroitin sulfate proteoglycan (MCSP), CCR8, TNF-alpha precursor, STEAP, mesothelin, A33 antigen, prostate stem cell antigen (PSCA), Ly-6, desmoglein 4, E-cadherin, neo-epitope, fetal acetylcholine receptor, CD25, CA19-9 marker, CA-125 marker, muellerian inhibitory substance (MIS) receptor type II, sialylated Tn antigen (sTn, TAG72), fibroblast activation antigen (FAP), endosialin, EGFRvIII, L6, SAS, CD63, TF-antigen, cora antigen, CD7, CD79b, CD22, Igα, Igβ, gp100, MT-MMPs, F19-antigen, CO-29 and EphA2.

In some embodiments, the TAA includes HER2, CD20, CD79b or CD47.

In some embodiments, the TAA-binding moiety comprises a heavy chain constant region of an antibody, and the heavy chain constant region of an antibody includes a heavy chain constant region from human IgG.

In some embodiments, the TAA-binding moiety comprises a heavy chain constant region of an antibody, wherein the heavy chain constant region of an antibody includes a heavy chain constant region from human IgG1, IgG2, IgG3 or IgG4.

In some embodiments, the TAA-binding moiety comprises a light chain constant region of an antibody, wherein the light chain constant region of an antibody includes a constant region of human Ig κ light chain or a constant region of human Ig λ light chain.

In another aspect, the present application provides an isolated nucleic acid molecule encoding the antigen-binding protein of the present application.

The nucleic acid molecule of the present application may be isolated. For example, it may be produced or synthesized by the following methods: (i) in vitro amplification, for example by polymerase chain reaction (PCR) amplification, (ii) clonal recombination, (iii) purification, for example, by fractional separation through restriction enzyme digestion and gel electrophoresis, or (iv) synthesis, for example, by chemical synthesis. In some embodiments, the isolated nucleic acid is a nucleic acid molecule produced by the recombinant DNA technology.

In the present application, the nucleic acid encoding the antibody and the antigen-binding fragment thereof may be prepared by a variety of methods known in the art. These methods include, but are not limited to, the overlap extension PCR using restriction fragments or using synthetic oligonucleotides. For specific operations, see Sambrook et al., Molecular Cloning, A Laboratory Manual, Cold Spring Harbor Laboratory Press, Cold Spring Harbor, N.Y, 1989; and Ausube et al., Current Protocols in Molecular Biology, Greene Publishing and Wiley-Interscience, New York N.Y., 1993.

In another aspect, the present application provides a vector comprising the isolated nucleic acid molecule of the present application. Each vector may comprise one or more of the nucleic acid molecules. In addition, the vector may also comprise other genes, for example, a marker gene that allows to select this vector in a suitable host cell and under a suitable condition. In addition, the vector may further comprise an expression control element that allows a coding region to be expressed correctly in a suitable host. Such a control element is well known to those skilled in the art, which, for example, may include a promoter, a ribosome binding site, an enhancer, and other control elements that regulate gene transcription or mRNA translation. In some embodiments, the expression control sequence is a regulatable element. A specific structure of the expression control sequence may vary depending on the function of the species or cell type, but generally includes a 5' non-transcribed sequence and 5' and 3' untranslated sequences, for example, a TATA box, a capped sequence and a CAAT sequence, which are involved in transcription and initiation of translation, respectively. For example, the 5' untranscribed expression control sequence may comprise a promoter region, and the promoter region may include a promoter sequence functionally linked to a nucleic acid for transcriptional control. The expression control sequence may also include an enhancer sequence or an upstream activator sequence. In the present application, suitable promoters may include, for example, promoters for SP6, T3, and T7 polymerases, human U6 RNA promoters, CMV promoters, and their artificial hybrid promoters (such as CMV), wherein a portion of a promoter may be fused with a portion of a promoter of another cellular protein (such as human GAPDH and glyceraldehyde-3-phosphate dehydrogenase) gene, and the promoter may or may not contain additional introns. The one or more nucleic acid molecules of the present application may be operably linked to the expression control element. The vector may include, for example, a plasmid, a cosmid, a virus, a bacteriophage, or other vectors commonly used in, for example, genetic engineering. For example, the vector is an expression vector.

In another aspect, the present application provides a host cell comprising the vector of the present application. In some embodiments, each type of or each host cell may comprise one or one type of the nucleic acid molecule or the vector of the present application. In some embodiments, each type of or each host cell may comprise a plurality of (for example, 2 or more) or a plurality of types of (for example, 2 or more types of) the nucleic acid molecule s or vectors of the present application. For example, the vector of the present application may be introduced into the host cell, for example, a eukaryotic cell, such as a cell from a plant, a fungal cell and a yeast cell. The vector of the present application may be introduced into the host cell by methods known in the art, such as electroporation, lipofectin transfection and lipofectamine transfection.

In another aspect, the present application provides a method for producing the antigen-binding protein of the present application. The method comprises culturing the host cell of the present application under a condition suitable for expressing the antigen-binding protein.

In some embodiments, the host cell is selected from the group consisting of a bacterial cell, a fungal cell, a plant cell, a mammalian cell and a virus.

In some embodiments, the bacterial cell is *Escherichia coli.*

In some embodiments, the fungal cell is a yeast cell.

In some embodiments, the mammalian cell is selected from the group consisting of CHO, NS0, BHK and HEK293 cells.

In some embodiments, the cell is a hybridoma cell.

In some embodiments, the hybridoma cell is selected from a mouse hybridoma cell, a rat hybridoma cell and a rabbit hybridoma cell.

In addition, the relevant sequences may also be synthesized using an artificial synthesis method, in particular when a fragment is short. Generally, a fragment with a very long sequence may be obtained by first synthesizing multiple small fragments, and then linking the small fragments. Then, the nucleic acid molecules may be introduced into various existing DNA molecules (or such as vectors) and cells known in the art.

The present application further provides vectors comprising the aforementioned appropriate nucleic acid molecules and appropriate promoters or control sequences. These vectors may be used for transforming appropriate host cells to enable them to express proteins. The host cells may be prokaryotic cells, such as bacterial cells; or lower eukaryotic cells, such as yeast cells; or higher eukaryotic cells, such as mammalian cells. For example, the animal cells may include CHO-S, CHO-K1, and HEK-293 cells.

The step of transforming the host cells with recombinant DNAs in the present application may be performed using technologies well known in the art. An obtained transformant may be cultured by a conventional method, and it expresses the polypeptide encoded by the nucleic acid molecule of the present application. The host cells are cultured in a conventional medium under suitable conditions according to their types. Generally, the host cells are cultured and transformed under conditions suitable for expressing the antigen-binding protein of the present application. Then, purification is performed using conventional immunoglobulin purification steps, for example conventional separation and purification means well known to those skilled in the art including protein A-Sepharose, hydroxyapatite chromatography, gel electrophoresis, dialysis, ion exchange chromatography, hydrophobic chromatography, molecular sieve chromatography and affinity chromatography to obtain the antigen-binding protein of the present application.

The resulting monoclonal antibody may be identified by conventional means. For example, the binding specificity of the monoclonal antibody may be determined by immunoprecipitation or in vitro binding assays, such as fluorescence activated cell sorting (FACS), radioimmunoassay (RIA), or enzyme-linked immunosorbent assay (ELISA).

In another aspect, the present application provides a pharmaceutical composition comprising the antigen-binding protein of the present application, the isolated nucleic acid molecule of the present application, the vector of the present application and/or the host cell of the present application, and optionally comprising a pharmaceutically acceptable carrier.

The pharmaceutical composition of the present application may comprise the antigen-binding protein of the present application at a safe and effective amount (e.g., 0.001-99 wt%, 0.01-90 wt%, or 0.1-80 wt%) and a pharmaceutically acceptable carrier. Such carriers may include, but are not limited to: saline, buffer, glucose, water, glycerol, ethanol, and combinations thereof. A pharmaceutical preparation should match the mode of administration. The pharmaceutical composition of the present application may be prepared into an injection form, for example, by a conventional method using normal saline or an aqueous solution containing glucose and other adjuvants. The pharmaceutical composition such as an injection and a solution should be manufactured under an aseptic condition. The administration amount of an active ingredient is a therapeutically effective amount. In addition, the antigen-binding protein of the present application may also be used together with other therapeutic agents.

The antigen-binding protein or pharmaceutical composition described herein may be formulated, dosed, and administered in line with good medical practices. The considerations in this case include the specific disorder being treated, the specific mammal being treated, the clinical condition of a single patient, the cause of the disorder, the site of agent delivery, the method of administration and other factors known to a medical practitioner. A therapeutic agent is not necessarily but optionally formulated and/or administered together with one or more agents that are currently used for preventing or treating the disorder in question. The effective amount of such additional agents depends on the amount of the therapeutic agent existing in the preparation, the type of disorder or treatment, and other factors discussed above. Generally, these agents may be used at any dose that is empirically/clinically determined to be appropriate and via any route that is empirically/clinically determined to be appropriate. Compared with a single therapy, the dose of the antibody administered in a combination therapy may be reduced. The progress of such a therapy may be easily monitored by conventional technologies.

In another aspect, the present application provides use of the antigen-binding protein of the present application, the isolated nucleic acid molecule of the present application, the vector of the present application, the host cell of the present application or the pharmaceutical composition of the present application in the manufacture of a medicament for treating a cancer.

In some embodiments, the cancer includes a solid tumor or a hematological neoplasm.

In some embodiments, the solid tumor is selected from the group consisting of prostate cancer, colorectal cancer, gastric cancer, clear cell renal cell carcinoma, bladder cancer, lung cancer, squamous cell carcinoma, glioma, breast cancer, renal cancer, neovascularization, clear cell renal cell carcinoma (CCRCC), pancreatic cancer, kidney cancer, uroepithelial carcinoma and liver-metastatic adenocarcinoma.

In some embodiments, the hematologic neoplasm is selected from the group consisting of acute myeloid leukemia (AML), myelodysplastic syndrome (MDS), acute lymphoblastic leukemia (ALL), diffuse large B-cell lymphoma (DLBCL), chronic myelogenous leukemia (CML) and blastic plasmacytoid dendritic cell neoplasm (BPDCN).

In another aspect, the present application provides a method for detecting CD3 and/or TAA in a sample using the antigen-binding protein of the present application, comprising contacting the antigen-binding protein with the sample and detecting or quantifying a conjugate formed by the bispecific antibody with CD3 and/or TAA.

In another aspect, the present application provides a method for treating a cancer in a subject in need thereof, comprising administering a therapeutically effective amount of the antigen-binding protein of the present application to the subject in need thereof for a time sufficient to treat the cancer.

In some embodiments, the cancer is a solid tumor or a hematological neoplasm.

In some embodiments, the solid tumor is selected from the group consisting of prostate cancer, colorectal cancer, gastric cancer, clear cell renal cell carcinoma, bladder cancer, lung cancer, squamous cell carcinoma, glioma, breast cancer, renal cancer, neovascularization, clear cell renal cell carcinoma (CCRCC), pancreatic cancer, kidney cancer, uroepithelial carcinoma and liver-metastatic adenocarcinoma.

In some embodiments, the hematologic neoplasm is selected from the group consisting of acute myeloid leukemia (AML), myelodysplastic syndrome (MDS), acute lymphoblastic leukemia (ALL), diffuse large B-cell lymphoma (DLBCL), chronic myelogenous leukemia (CML) and blastic plasmacytoid dendritic cell neoplasm (BPDCN).

In some embodiments, the antibody is administered in combination with a second therapeutic agent.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to modify the specific embodiments disclosed without departing from the spirit and scope of the present application. Accordingly, the accompanying drawings and the description in the specification of the present application are merely for an exemplary but not limiting purpose.

### BRIEF DESCRIPTION OF DRAWINGS

The specific features of the invention according to the present application are set forth in the appended claims. The characteristics and advantages of the invention according to the present application can be better understood by reference to the exemplary embodiments described in detail below and the accompanying drawings. Brief descriptions of the accompanying drawings are as follows.
FIG. 1 shows the analysis of purification of anti-CD3 antibodies.
FIG. 2 shows the yield and concentration of purified anti-CD3 antibodies.
FIG. 3 shows details of the CD3E/G antigen used in the ELISA.
FIGs. 4A to 4C show the results of the binding of the CD3 antibody to the antigen as detected by ELISA.
FIG. 5 shows EC₅₀ values of the binding of the CD3 antibody to the antigen as detected by ELISA.
FIG. 6 (FIGs 6-1 to 6-7) shows the results of the binding affinity assay of CD3 antibody to Jurkat cells.

### DETAILED DESCRIPTION

The embodiments of the present disclosure will be illustrated by specific examples below, and those skilled in the art can easily understand other advantages and effects of the present application from the content disclosed in the specification.

### Definition of Terms

Unless otherwise indicated, the implementation of the present disclosure will adopt conventional technologies of molecular biology (including recombinant technology), microbiology, cell biology, biochemistry and immunology, which shall fall within the technical scope of the art.

So that the present invention may be more readily understood, certain scientific and technical terms are specifically defined below. Unless specifically defined elsewhere herein, all scientific and technical terms used herein have the meaning commonly understood by those of ordinary skill in the art to which the present application belongs. For definitions and terms in the art, those skilled can refer to Current Protocols in Molecular Biology (Ausubel). The abbreviations of amino acid residues are the standard 3-letter and/or 1-letter codes used in the art to refer to one of the 20 commonly used L-amino acids. As used herein, including the claims, the singular forms of words include their corresponding plural forms unless the context clearly dictates otherwise.

In the present disclosure, the term "about" used in combination with a numerical value is intended to encompass the numerical values in a range from a lower limit less than the specified numerical value by 5% to an upper limit greater than the specified numerical value by 5%.

In the present disclosure, the term "and/or" should be understood to refer to any one of the options or any two or more of the options.

In the present disclosure, the term "CD3" generally refers to a portion of T cell receptor complex, consisting of three different chains: CD3ε, and CD3δ and CD3γ. The clustering of CD3 on T cells resulted from, e.g. the fixation effect of anti-CD3 antibodies to CD3, leads to activation of T cells in a manner similar to T cell receptor-mediated activation, but independent of the specificity of the TCR clone. Most anti-CD3 antibodies can recognize the CD3ε chain. The term refers to any native CD3 from any vertebrate (including mammals such as primates (e.g., human)) and rodents (e.g., mice and rats). Unless otherwise specified, the term encompasses "full-length" unprocessed CD3 as well as any form of CD3 or any fragment thereof produced by intracellular processing. The term also includes variants of naturally occurring CD3, e.g., splice variants or allelic variants. In a preferred embodiment, CD3 refers to full length CD3 or fragments thereof (e.g., mature fragments lacking a signal peptide) from human and cynomolgus monkeys. In a preferred embodiment, CD3 refers to full length CD3 or fragments thereof (e.g., mature fragments lacking a signal peptide) from mice/rats.

In the present disclosure, the term "percent (%) of amino acid sequence identity" or simply "identity" is defined as the percentage of amino acid residues in a candidate amino acid sequence that are identical to those in a reference amino acid sequence after aligning amino acid sequences (with gaps introduced if necessary) to achieve maximum percent of sequence identity without considering any conservative replacement as part of sequence identity. Various methods in the art can be employed to perform sequence alignment so as to determine the percent of amino acid sequence identity, for example, using computer software available to the public, such as software BLAST, BLAST-2, ALIGN, and MEGALIGN (DNASTAR). Those skilled in the art can determine suitable parameters for measuring alignment, including any algorithm required to obtain maximum alignment of the full length of the aligned sequences.

In the present disclosure, the term "immune response" generally refers to the action of producing soluble macromolecules (including antibodies, cytokines, and complement) by, such as lymphocytes, antigen-presenting cells, phagocytes, granulocytes and liver, which results in the selective damage, destruction, or elimination of pathogens, cells or tissues infected with pathogens, cancerous cells, or normal human cells or tissues in the case of autoimmunity or pathological inflammation.

In the present disclosure, the term "signal transduction pathway" or "signaling activity" refers to a biochemical causal relationship generally initiated by a protein-protein interaction such as binding of a growth factor to a receptor, resulting in transmission of a signal from one portion of a cell to another portion of a cell. In general, the transmission involves specific phosphorylation of one or more tyrosine, serine, or threonine residues of one or more proteins in the series of reactions causing signal transduction. Penultimate processes typically include nuclear events, resulting in a change in gene expression.

In the present disclosure, the term "activity" or "biological activity", or the term "biological property" or "biological characteristic" are used interchangeably herein, including but not limited to epitope/antigen affinity and specificity, ability of neutralizing or antagonizing CD3 activity in vivo or in vitro, IC₅₀, in vivo stability of the antibody, and immunogenic properties of the antibody. Other identifiable biological properties or characteristics of antibodies well known in the art include, for example, cross-reactivity (i.e., cross-reactivity typically with a non-human homologue of the targeted peptide or with other proteins or tissues), and the ability to maintain high expression levels of the protein in mammalian cells. The aforementioned properties or characteristics are observed, determined or assessed using techniques well known in the art, including, but not limited to, ELISA, FACS, BIACORE plasmon resonance assays, unrestricted in vitro or in vivo neutralization assays, receptor binding, production and/or secretion of cytokines or growth factors, signal transduction, and immunohistochemistry of tissue sections of different origins, including human, primate, or any other origin.

In the present application, the term "antigen-binding protein" generally refers to a protein comprising a moiety that can bind to an antigen, and optionally a scaffold or skeleton portion that allows the moiety that can bind to the antigen to adopt a conformation that promotes the binding of the antigen-binding protein to the antigen. Examples of the antigen-binding protein include, but are not limited to, antibodies, antigen-binding fragments (Fab, Fab', F(ab)₂, Fv fragments, F(ab')₂, scFv, di-scFv and/or dAb), immunoconjugates, multi-specific antibodies (for example, bispecific antibodies), antibody fragments, antibody derivatives, antibody analogs and fusion proteins, as long as they exhibit the desired antigen-binding activity. The "isolated antigen-binding protein" of the present disclosure may comprises a moiety that can bind to an antigen, and optionally a scaffold or skeleton portion that allows the moiety that can bind to the antigen to adopt a conformation that promotes the binding of the antigen-binding protein to the antigen.

In the present disclosure, the term "antibody" refers to any form of antibodies having a desired bioactivity. Thus, it is used in the broadest sense and specifically includes, but is not limited to, monoclonal antibodies (including full length monoclonal antibodies), polyclonal antibodies, multi-specific antibodies (e.g., bispecific antibodies), humanized antibodies, fully human antibodies, chimeric antibodies, and camelized single domain antibodies. The basic structural unit of antibody is known to comprise a tetramer. Each tetramer comprises two identical pairs of polypeptide chains, and each pair of polypeptide chains has one "light" chain (about 25 kDa) and one "heavy" chain (about 50-70 kDa). The amino-terminal portion or fragment of each chain may comprise a variable region of about 100-110 or more amino acids primarily responsible for antigen recognition. The carboxyl-terminal portion or fragment of each chain may define a constant region primarily responsible for effector function. Human light chains are generally classified to be κ and λ light chains. Furthermore, human heavy chains are generally classified to be m, δ, y, α and ε. The antibodies are classified to be isotypes of IgM, IgD, IgG, IgA, and IgE. Within the light and heavy chains, the variable and constant regions are connected by a "J" region of about 12 or more amino acids, and the heavy chain also comprises a "D" region of about 10 more amino acids. See generally, Fundamental Immunology, Chap. 7 (Paul, W. ed., 2nd edition. Raven Press, N.Y.(1989)).

In the present disclosure, the term "isolated antibody" refers to the purified state of a binding-compound, and in this case means that the molecule is substantially free of other biomolecules, such as nucleic acids, proteins, lipids, sugars, and other substances such as cell debris and growth media. The term "isolated" does not mean the complete absence of the above substances or the absence of water, buffers, or salts, unless they are present in an amount that significantly interferes with the experimental or therapeutic use of the binding-compound described herein.

In the present disclosure, the term "monoclonal antibody" refers to an antibody obtained from a substantially homogeneous population of antibodies, i.e., the antibodies making up the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristics of an antibody obtained from a substantially homogeneous antibody population, and is not to be construed as producing the antibody by any particular method.

In the present disclosure, the term "bispecific antibody" generally refers to an artificially designed antibody, which is composed of components of two different antigen binding sites and can bind to two different antigen binding sites simultaneously.

In the present disclosure, the term "full length antibody" generally refers to an immunoglobulin molecule comprising four peptide chains when naturally occurring, wherein two weight (H) chains (about 50-70 kDa in full length) and two light (L) chains (about 25 kDa in full length) are linked by disulfide bonds. Each heavy chain is composed of a heavy chain variable region (abbreviated herein as VH) and a heavy chain constant region (abbreviated herein as CH). The heavy chain constant region consists of three domains, CH1, CH2 and CH3. Each light chain is composed of a light chain variable region (abbreviated herein as VL) and a light chain constant region. The light chain constant region consists of one domain CL. The VH and VL regions can be further subdivided into complementarity determining regions (CDRs) with hypervariability and more conservative framework regions (FRs) interspaced therewith. Each VH or VL region is composed of 3 CDRs and 4 FRs in the following order from amino to carboxyl terminus: FR1, CDR1, FR2, CDR2, FR3, CDR3, FR4. The variable region of the heavy and light chains contains a binding domain that interacts with an antigen. The constant region of an antibody can mediate the binding of the immunoglobulin to host tissues or factors, including various cells of the immune system (e.g., effector cells) and the first component (Clq) of the classical complement system.

In the present disclosure, the term "antigen-binding fragment" of an antibody ("parent antibody") includes a fragment or derivative of an antibody, typically comprising at least one fragment of an antigen-binding region or variable region (e.g., one or more CDRs) of the parental antibody, which retains at least some binding specificity of the parent antibody. Examples of binding fragments of an antibody include, but are not limited to, Fab, Fab', F(ab')₂, Fv fragments, diabodies, linear antibodies, single chain antibody molecules (e.g., sc-Fv), nanobodies and multi-specific antibodies formed by antibody fragments. When the antigen binding activity is represented on a basis of molar concentration, the binding fragment or derivative typically retains at least 10% of the antigen binding activity of the parent antibody. Preferably the binding fragment or derivative retains at least 20%, 50%, 70%, 80%, 90%, 95% or 100% or more of the antigen binding activity of the parent antibody. It is also contemplated that antigen-binding fragments of antibodies can include conservative or non-conservative amino acid substitutions that do not significantly alter their biological activity (referred to as "conservative variants" or "function-conservative variants" of antibodies). The term "binding compound" refers to both antibodies and binding fragments thereof.

In the present disclosure, the term "single chain Fv" or "scFv" antibody generally refers to an antibody fragment comprising the VH and VL domains of the antibody, wherein these domains are present in a single polypeptide chain. A scFv polypeptide also generally comprises a polypeptide linker between the VH and the VL domains that enables the scFv to form the desired structure for antigen binding.

In the present disclosure, the term "complementarity determining region (CDR)" refers to an antibody region that binds to an antigen. CDRs can be defined using various delineations such as Kabat (Wu et al., J Exp Med, 1970, vol. 132, pp. 211-250) (Kabat et al., Sequences of Proteins of Immunological Interest, 5th Ed. Public Health Service, National Institutes of Health, Bethesda, Md., 1991), Chothia (Chothia et al., J Mol Biol, 1987, vol. 196, pp. 901-917), IMGT (Lefranc et al., Dev Comp Immunol, 2003, vol. 27, pp. 55-77) and AbM (Martin and Thornton, J Bmol Biol, 1996, vol. 263, pp. 800-815). The correspondence between the various delineations and variable region numbering are described (see e.g., Lefranc et al., Dev Comp Immunol, 2003, vol. 27, pp. 55-77; Honegger and Pluckthun, J Mol Biol, 2001, vol. 309, pp. 657-670; international ImMunoGeneTics (IMGT) information system; and Web resource: http://www.imgt.org). Available programs such as abYsis of UCL Business PLC can be used to delineate CDRs. The terms "CDR", "HCDR1", "HCDR2", "HCDR3", "LCDR1", "LCDR2" and "LCDR3" as used herein include CDRs defined by any of the methods described above (Kabat, Chothia, IMGT or AbM), unless otherwise explicitly stated in the specification. For example, the CDR region of the present application can be defined using the Kabat numbering system.

In the present disclosure, the term "Fc", "Fc region" or "Fc fragment" generally refers to a polypeptide consisting of CH2 and CH3 domains of IgA, IgD or IgG, or consisting of CH2, CH3, and CH4 domains of IgE or IgM, through a hinge region. Although the decomposition of the Fc fragment is variable, the heavy chain Fc fragment of human IgG usually refers to the polypeptide fragment from A231 to its carboxyl terminus.

In the present disclosure, the term "hinge region" generally refers to a polypeptide chain located between CH1 and CH2 in an antibody, which is rich in proline and is easy to stretch and bend. The commonly recognized IgG hinge region is a polypeptide chain composed of amino acid residues from position 216 to position 230.

In the present disclosure, the term "domain antibody" generally refers to an immune-functional immunoglobulin fragment that consist of only a heavy chain variable region or a light chain variable region. In some cases, two or more VH regions are covalently linked to a peptide linker to form a bivalent domain antibody. The two VH regions of the bivalent domain antibody may target the same or different antigens.

In the present disclosure, the term "bivalent antibody" comprises two antigen-binding sites. In some cases, the two binding sites have the same antigen specificity. However, a bivalent antibody may be bispecific.

In the present disclosure, the term "diabody" generally refers to a small antibody fragment having two antigen-binding sites, which comprises a heavy chain variable domain (VH) linked to a light chain variable domain (VL) in one polypeptide chain (VH-VL or VL-VH). By using a linker that is too short to pair between two domains of one chain, the domains are forced to pair with the complementary domains of the other chain to form two antigen-binding sites.

In the present disclosure, the term "chimeric antibody" generally refers to an antibody having the variable domains of a first antibody and the constant domains of a second antibody, wherein the first and second antibodies are from different species. Typically, the variable domain is obtained from an antibody of for example a rodent ("parent antibody"), and the constant domain sequence is obtained from a human antibody, such that the resulting chimeric antibody is less likely to induce an adverse immune response in a human subject as compared to the parent rodent antibody.

In the present disclosure, the term "humanized antibody" generally refers to an antibody from a non-human (e.g., mouse) immunoglobulin, which has been engineered to contain minimal non-human (e.g., mouse) sequences. Typically, humanized antibodies are human immunoglobulins whose residues of the complementary determining regions (CDRs) are replaced by the residues of CDRs from a non-human species (e.g., mouse, rat, rabbit, or hamster) that have the desired specificity, affinity, and capability (Jones et al., Nature, 321:522-525(1986); Riechmann et al., Nature, 332:323-327(1988); Verhoeyen et al., Science, 239:1534-1536(1988)). In some instances, the residues of Fv framework region (FW) of a human immunoglobulin are replaced with the corresponding residues of an antibody from a non-human species that has the desired specificity, affinity, and capability.

In the present disclosure, the term "fully human antibody" generally refers to an antibody that contains only human immunoglobulin protein sequences. A fully human antibody may contain mouse carbohydrate chains if it is produced in a mouse, in a mouse cell, or in a hybridoma from a mouse cell. Likewise, a "mouse antibody" refers to an antibody that contains only mouse immunoglobulin sequences. Alternatively, a fully human antibody may contain rat carbohydrate chains if it is produced in a rat, in a rat cell, or in a hybridoma from a rat cell. Likewise, a "rat antibody" refers to an antibody that contains only rat immunoglobulin sequences.

In the present disclosure, "isotype" antibodies generally refer to an antibody class (e.g., IgM, IgE, IgG such as IgG1, IgG2 and IgG4) that is provided by the heavy chain constant region genes. Isotype also includes the modified forms of one of these classes, where modifications have been made to alter the Fc function, for example, to enhance or reduce effector functions or binding to Fc receptors.

In the present disclosure, the term "epitope" generally refers to an antigen region to which an antibody binds. Epitopes can be formed by contiguous amino acids or noncontiguous amino acids juxtaposed by tertiary folding of a protein.

In the present disclosure, "affinity" or "binding affinity" generally refers to intrinsic binding affinity reflecting an interaction between members of a binding. The affinity of a molecule X for its partner Y can generally be represented by the equilibrium dissociation constant (KD), and the equilibrium dissociation constant is the ratio of the dissociation and association rate constants (kdis and kon, respectively). Affinity can be measured by common methods known in the art. One particular method for measuring affinity is the ForteBio binding kinetic assay herein.

In the present disclosure, the term "not binding" to protein or cell generally refers to not binding to protein or cell or not binding thereto with high affinity, i.e. binding to protein or cell with KD of 1.0×10⁻⁶ M or higher, more preferably 1.0×10⁻⁵ M or higher, more preferably 1.0×10⁻⁴ M or higher, more preferably 1.0×10⁻³ M or higher, more preferably 1.0×10⁻² M or higher.

In the present disclosure, the term "high affinity" for IgG antibodies refers to KD for antigen of 1.0×10⁻⁶ M or less, preferably 5.0×10⁻⁸ M or less, more preferably 1.0×10⁻⁸ M or less, more preferably 5.0×10⁻⁹M or less, more preferably 1.0×10⁻⁹ M or less. For other antibody isoforms, "high affinity" binding may vary. For example, "high affinity" binding for IgM isoforms refers to KD of 10⁻⁶ M or less, preferably 10⁻⁷ M or less, more preferably 10⁻⁸ M or less.

In the present disclosure, the term "antibody-dependent cytotoxicity", "antibody-dependent cell-mediated cytotoxicity" or "ADCC" refers to cell-mediated immune defense in which immune system effector cell actively lyses target cells whose cell membrane surface antigen binds to the antibody, for example cancer cells.

In the present disclosure, the term "complement dependent cytotoxicity" or "CDC" generally refers to effector function of IgG and IgM antibodies, which elicit classical complement pathway when they bind to surface antigens, including formation of membrane attack complex and lysis of target cells.

In the present disclosure, the term "nucleic acid" or "polynucleotide" generally refers to deoxyribonucleic acids (DNA) or ribonucleic acids (RNA) and polymers thereof in single-or double-stranded form. Unless expressly limited, the term includes nucleic acids containing known analogs of natural nucleotides that have similar binding properties as the reference nucleic acid and are metabolized in a manner similar to the naturally occurring nucleotides (see U.S. Patent No. 8,278,036 by Kariko et al., which discloses an mRNA molecule in which uridine is replaced by pseudouridine, a method for synthesizing the mRNA molecule and a method for delivering a therapeutic protein in vivo). Unless otherwise noted, a particular nucleic acid sequence also implicitly encompasses conservatively modified variants thereof (e.g., degenerate codon substitutions), alleles, orthologs, SNPs, and complementary sequences as well as sequences explicitly indicated. Specifically, degenerate codon substitutions can be achieved by generating sequences in which the third position of one or more selected (or all) codons is substituted with mixed-base and/or deoxyinosine residues (Batzer et al., Nucleic Acid Res. 19:5081(1991); Ohtsuka et al., J. Biol. Chem. 260:2605-2608(1985); and Rossolini et al., Mol. Cell. Probes. 8:91-98(1994)).

In the present disclosure, "construct" generally refers to any recombinant polynucleotide molecule (such as a plasmid, cosmid, virus, autonomously replicating polynucleotide molecule, bacteriophage, and linear or circular single-stranded or double-stranded DNA or RNA polynucleotide molecule), which is from any source, capable of integrating with the genome or autonomously replicating to form the polynucleotide molecules in which one or more polynucleotide molecules have been linked in a functionally operative manner (i.e., operably linked). Recombinant constructs generally comprise the polynucleotide of the present invention operably linked to transcription initiation regulatory sequences that will direct the transcription of the polynucleotide of the present invention in a host cell. Both heterologous and non-heterologous (i.e., endogenous) promoters may be used to direct the expression of the polynucleotide of the present invention.

In the present disclosure, "vector" generally refers to any recombinant polynucleotide construct, and the construct may be used for the purpose of transformation (i.e. to introduce heterologous DNA into a host cell). One type of vector is a "plasmid", which refers to a circular double stranded DNA loop. Additional DNA fragments may be ligated into this loop. Another type of vector is a viral vector, wherein additional DNA fragments may be ligated into viral genome. Certain vectors are capable of autonomously replicating in a host cell into which they are introduced (e.g., bacterial vectors having a bacterial replication origin and episomal mammalian vectors). Other vectors (e.g., non-episomal mammalian vectors) are integrated into the genome of a host cell upon introducing into a host cell, and thus are replicated along with the host genome. Moreover, certain vectors are capable of directing expression of operably linked genes. Such vectors are referred to herein as "expression vectors".

In the present disclosure, the term "expression vector" generally refers to a nucleic acid molecule capable of replicating and expressing a gene of interest when transformed, transfected or transduced into a host cell. An expression vector comprises one or more phenotypic selectable markers and an origin of replication to ensure maintenance of vector and to provide amplification in host if necessary.

In the present disclosure, "activation", "stimulation" and "treatment" for a cell or receptor can have the same meaning, e.g. a cell or receptor is activated, stimulated or treated with a ligand, unless otherwise or clearly dictated by context. "Ligand" includes natural and synthetic ligands, for example, cytokines, cytokine variants, analogs, mutant proteins, and antibody-derived binding compounds. "Ligand" also includes small molecules, for example, peptidomimetics of cytokines and peptidomimetics of antibodies. "Activation" may refer to activation of a cell regulated by internal mechanisms as well as external or environmental factors. "Response/reaction", e.g., a response of a cell, tissue, organ, or organism, including a change in biochemical or physiological behavior (such as concentration, density, adhesion or migration, rate of gene expression, or state of differentiation within a biological compartment), wherein the change is related to activation, stimulation, or treatment, or related to an internal mechanism such as genetic programming.

In the present disclosure, the term "treatment" of any disease or disorder refers, in one embodiment, to ameliorating the disease or disorder (i.e., slowing or preventing or reducing the progression of the disease or at least one of the clinical symptoms thereof). In another embodiment, "treatment" refers to alleviating or ameliorating at least one physical parameter, including those physical parameters that may not be discernible by the patient. In another embodiment, "treatment" refers to modulating a disease or disorder, either physically (e.g., stabilization of a discernible symptom), physiologically (e.g., stabilization of a physical parameter), or both. Methods for assessing treatment and/or prevention of a disease are generally known in the art, unless specifically described herein.

In the present disclosure, a "subject" includes any human or non-human animal. The term "non-human animal" includes all vertebrates, e.g., mammals and non-mammals, such as non-human primates, sheep, dogs, cats, horses, cows, chickens, amphibians and reptiles. As used herein, the term "cyno" or "cynomolgus monkey" refers to cynomolgus monkey.

In the present disclosure, the administration "in combination with" one or more additional therapeutic agents includes simultaneous (co-) administration and consecutive administration in any order.

In the present disclosure, a "therapeutically effective amount," "therapeutically effective dose," and "effective amount" generally refer to an amount effective to prevent or ameliorate one or more symptoms of diseases or conditions, or the development of the diseases or conditions when the antigen-binding protein of the present disclosure is administered alone or in combination with additional therapeutic agents to a cell, tissue, or subject. A therapeutically effective dose also refers to an amount of the antibody or antigen binding fragment thereof sufficient to cause amelioration of symptoms, such as an amount to treat, cure, prevent, or ameliorate relevant medical conditions or to increase the rate of treatment, cure, prevention, or amelioration of such conditions. When an active ingredient is administered alone to a subject, a therapeutically effective dose refers to the ingredient alone. When active ingredients are administered in combination, a therapeutically effective dose refers to the combined amount of the active ingredients that causes a therapeutic effect, whether administered in combination, sequentially or simultaneously. An effective amount of a therapeutic agent will result in an increase in a diagnostic criterion or parameter by at least 10%, usually at least 20%, preferably at least about 30%, more preferably at least 40%, most preferably at least 50%.

In the present disclosure, the terms "cancer" and "cancerous" generally refer to or describe the physiological condition in mammals that is typically characterized by uncontrolled cell growth. The terms include benign and malignant cancers as well as dormant tumors or micrometastatses. Examples of cancer include but are not limited to, carcinoma, lymphoma, blastoma, sarcoma, and leukemia. More particular examples of such cancers include but are not limited to, squamous cell cancer, lung cancer (including small-cell lung cancer, non-small cell lung cancer, adenocarcinoma of the lung, and squamous carcinoma of the lung), peritoneal cancer, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's macroglobulinemia), chronic lymphocytic leukemia (CLL), acute lymphoblastic leukemia (ALL), hairy cell leukemia, chronic myeloblastic leukemia, and post-transplant lymphoproliferative disorder (PTLD) and abnormal vascular proliferation associated with phakomatoses, edema (such as those associated with brain tumors), and Meigs syndrome.

Without intending to be bound by any theory, the examples hereinafter are merely intended to illustrate the antigen-binding protein, production method and use thereof of the present disclosure, rather than to limit the scope of the invention of the present disclosure.

### Examples

### Example 1 Production of anti-CD3 monoclonal antibody

### 1.1. Construction of plasmid of anti-CD3 monoclonal antibody

The anti-CD3 sequence was modified, fused with the constant region of IgG1 and the constant region of the kappa chain respectively, and cloned into the expressing frame delimited by HindIII and NotI in a pTT5 vector to obtain a recombinant plasmid. The constructed plasmid was identified by sequencing to obtain recombinant plasmids having the correct sequence. These plasmids were extracted using Qiagen Midi Plasmid Extraction Kit.

### 1.2. Transient expression of anti-CD3 antibody

In this example, anti-CD3 antibody was produced using ExpiCHO expression system. Cryopreserved ExpiCHO-S^{™} cells were thawed and cultured (ExpiCHO^{™} Expression Medium). The cells were allowed to grow at a normal rate and were passaged 2 or more times before transfection.

One day before the transfection, the cell was adjusted to a density of 3×10⁶ to 4x10⁶ cells/ml and grew to a density of 7×10⁶ to 10×10⁶ cells/ml the next day.

On the day of the transfection, the cells were diluted to a density of 6×10⁶ cells/ml with fresh ExpiCHO^{™} Expression Medium pre-warmed at 37°C. ExpiFectamine^{™} CHO Reagent and plasmid DNA (heavy chain: light chain = 1:1) were diluted respectively using cold OptiPRO^{™} medium. The diluted ExpiFectamine^{™} CHO Reagent was added to the diluted plasmid and mixed evenly by inversion up and down to obtain a mixture of ExpiFectamine^{™} CHO and plasmid DNA. After 5 min of incubation at room temperature, the mixture was slowly added to ExpiCHO-S cell suspension, mixed evenly and cultured in a 37°C, 8% CO₂ incubator. One day after the transfection, 120 µL of ExpiCHO^{™} Enhancer and 4.8 mL of ExpiCHO^{™} Feed were added to the cell suspension, and the cells were cultured at 37°C for expression.

Seven days after the transfection, the cell suspension was centrifuged, and the supernatant was collected for antibody purification.

### 1.3. Purification and analysis of anti-CD3 monoclonal antibody

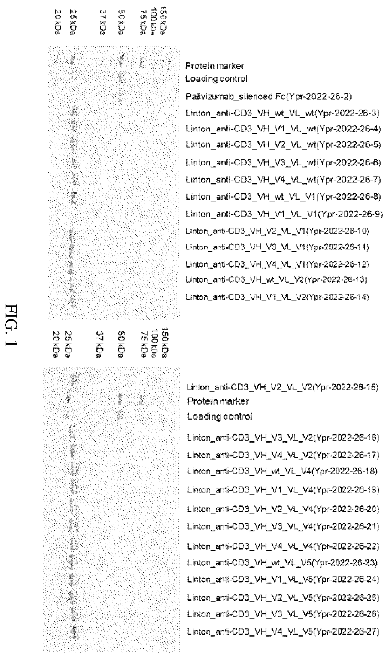

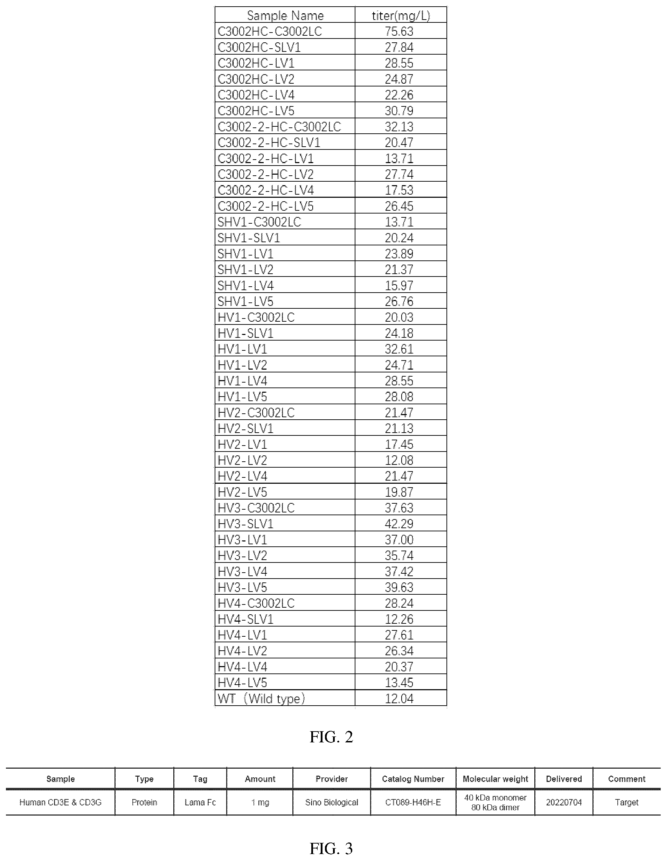

The supernatant obtained from the anti-CD3 antibody-expressing cell culture in the previous step was purified using Ni-NTA. The proteins in the supernatant were captured, washed using 20 mM imidazole in PBS (pH 7.4) and eluted with 200 mM imidazole in PBS (pH 7.4). The solution of the eluted proteins was ultrafiltered to replace the sample buffer with PBS (pH 7.4). The purified proteins were obtained and analyzed by SDS-PAGE (FIG. 1). The final yield was measured at OD280, as shown in FIG. 2.

The names of the antibody samples obtained are shown below.

| Number | Sample name | Original name | SEQ ID NO: |
|---|---|---|---|
| 1 | C3002HC-C3002LC | | 37 and 40 |
| 2 | C3002HC-SLV1 | | 37 and 39 |
| 3 | C3002HC-LV1 | | 37 and 20 |
| 4 | C3002HC-LV2 | | 37 and 21 |
| 5 | C3002HC-LV4 | | 37 and 22 |
| 6 | C3002HC-LV5 | | 37 and 23 |
| 7 | C3002-2HC-C3002LC | | 38 and 40 |
| 8 | C3002-2HC-SLV1 | | 38 and 39 |
| 9 | C3002-2HC-LV1 | | 38 and 20 |
| 10 | C3002-2HC-LV2 | | 38 and 21 |
| 11 | C3002-2HC-LV4 | | 38 and 22 |
| 12 | C3002-2-HC-LV5 | | 38 and 23 |
| 13 | SHV1-C3002LC | | 36 and 40 |
| 14 | SHV1-SLV1 | | 36 and 39 |
| 15 | SHV1-LV1 | | 36 and 20 |
| 16 | SHV1-LV2 | | 36 and 21 |
| 17 | SHV1-LV4 | | 36 and 22 |
| 18 | SHV1-LV5 | | 36 and 23 |
| 19 | HV1-C3002LC | | 15 and 40 |
| 20 | HV1-SLV1 | | 15 and 39 |
| 21 | HV1-LV1 | Linton_anti-CD3_VH_V1_VL_V1 | 15 and 20 |
| 22 | HV1-LV2 | Linton_anti-CD3_VH_V1_VL_V2 | 15 and 21 |
| 23 | HV1-LV4 | Linton_anti-CD3_VH_V1_VL_V4 | 15 and 22 |
| 24 | HV1-LV5 | Linton_anti-CD3_VH_V1_VL_V5 | 15 and 23 |
| 25 | HV2-C3002LC | | 16 and 40 |
| 26 | HV2-SLV1 | | 16 and 39 |
| 27 | HV2-LV1 | Linton_anti-CD3_VH_V2_VL_V1 | 16 and 20 |
| 28 | HV2-LV2 | Linton_anti-CD3_VH_V2_VL_V2 | 16 and 21 |
| 29 | HV2-LV4 | Linton_anti-CD3_VH_V2_VL_V4 | 16 and 22 |
| 30 | HV2-LV5 | Linton_anti-CD3_VH_V2_VL_V5 | 16 and 23 |
| 31 | HV3-C3002LC | | 17 and 40 |
| 32 | HV3-SLV1 | | 17 and 39 |
| 33 | HV3-LV1 | Linton_anti-CD3_VH_V3_VL_V1 | 17 and 20 |
| 34 | HV3-LV2 | Linton_anti-CD3_VH_V3_VL_V2 | 17 and 21 |
| 35 | HV3-LV4 | Linton_anti-CD3_VH_V3_VL_V4 | 17 and 22 |
| 36 | HV3-LV5 | Linton_anti-CD3_VH_V3_VL_V5 | 17 and 23 |
| 37 | HV4-C3002LC | | 18 and 40 |
| 38 | HV4-SLV1 | | 18 and 39 |
| 39 | HV4-LV1 | Linton_anti-CD3_VH_V4_VL_V1 | 18 and 20 |
| 40 | HV4-LV2 | Linton_anti-CD3_VH_V4_VL_V2 | 18 and 21 |
| 41 | HV4-LV4 | Linton_anti-CD3_VH_V4_VL_V4 | 18 and 22 |
| 42 | HV4-LV5 | Linton_anti-CD3_VH_V4_VL_V5 | 18 and 23 |

### Example 2: Analysis of binding of CD3 antibody to CD3E & CD3G heterodimer protein

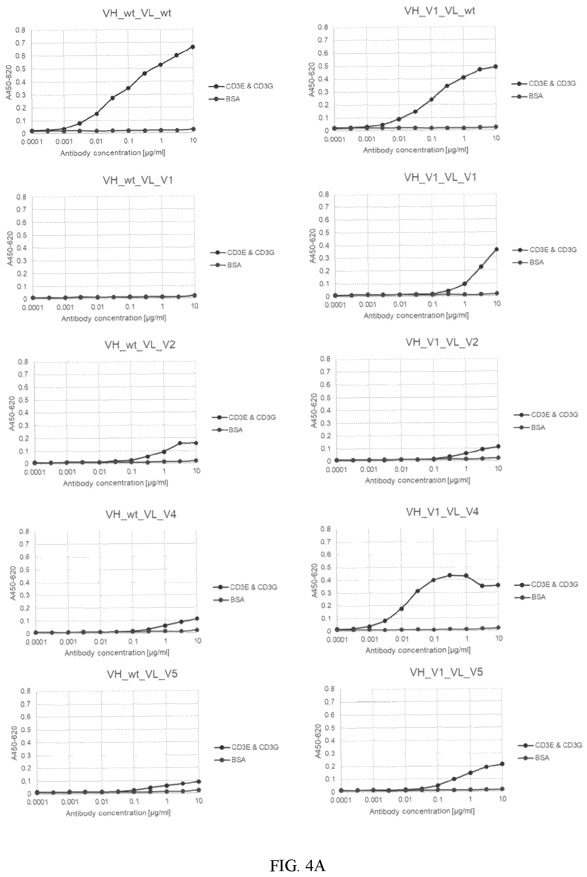

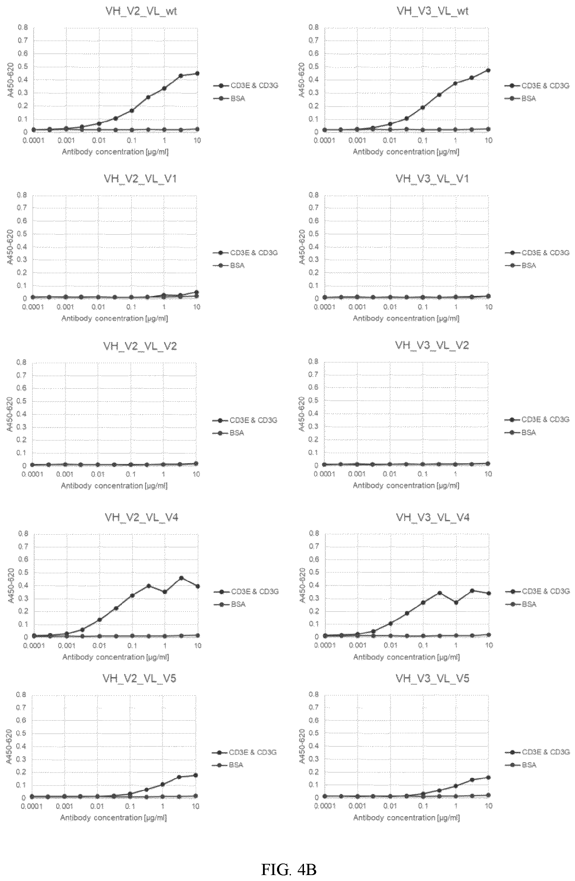

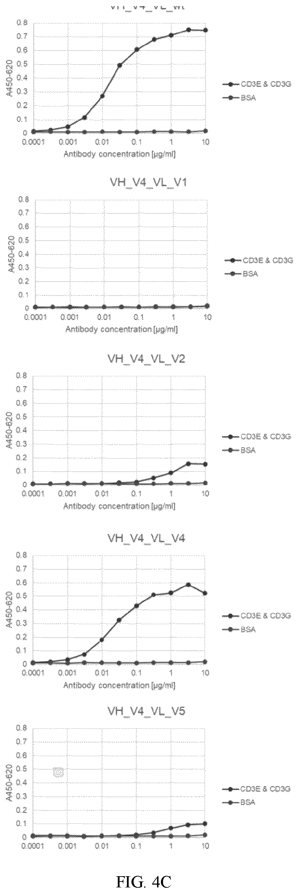

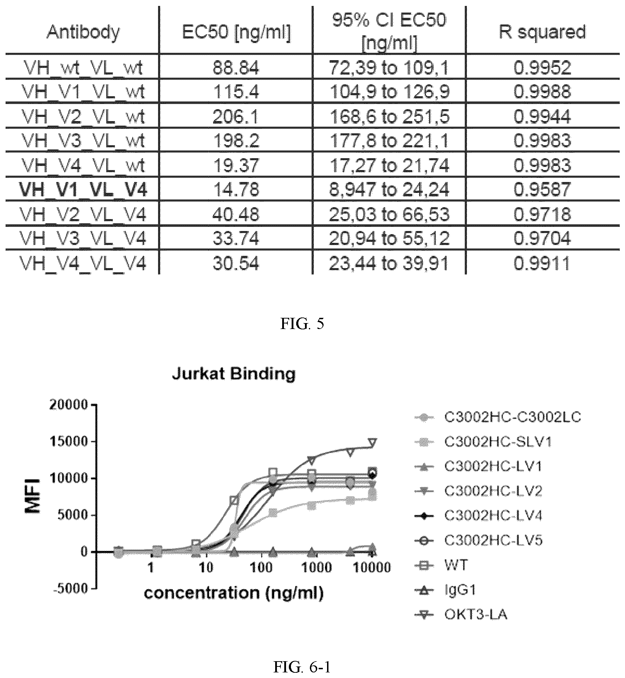

An ELISA plate was coated with CD3E & CD3G heterodimer protein (as shown in FIG. 3) at 100ng/well at 4°C overnight. The next day, the plate was blocked with a blocking solution (3% BSA in 1×PBS) for 2 h, washed with a washing solution and drained. The plate was then added with gradiently diluted humanized CD3 antibodies, and incubated at 37°C for 1 h. The reaction solution was discarded, and the plate was washed and drained. The plate was then added with diluted detection antibody (THE^{™} His Tag Antibody [HRP]) at 100 µL per well and incubated at 37°C for 1 h. The detection antibody was discarded, and the plate was washed and drained. The plate was then added with a single-component TMB color developing solution returned to room temperature at 100 µL per well, and the color development was performed at 37°C for 10 min. After the color was developed, a stop solution was added to the plate at 100 µL per well to terminate the color development reaction. The absorbance was read at 450 nm using a microplate reader. The read data were processed and presented using GraphPad Prism 8, as shown in FIGs. 4A to 4C. The EC₅₀ values for some of these antibodies are shown in FIG. 5.

### Example 3 Binding affinity assay of CD3 antibody to Jurkat cells

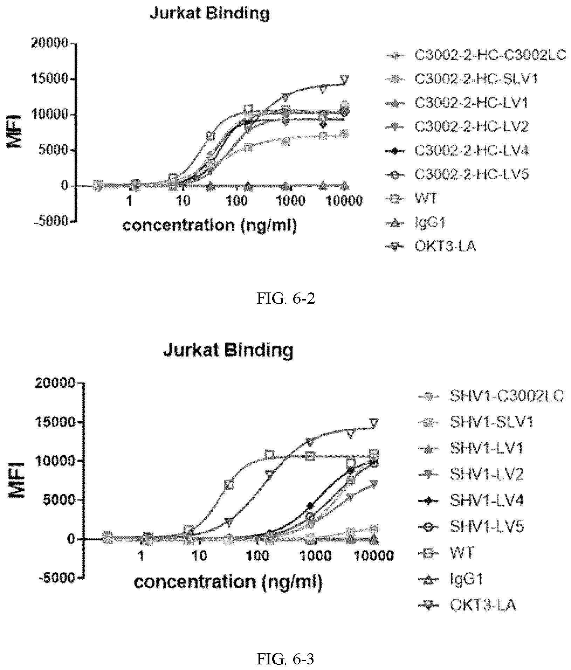

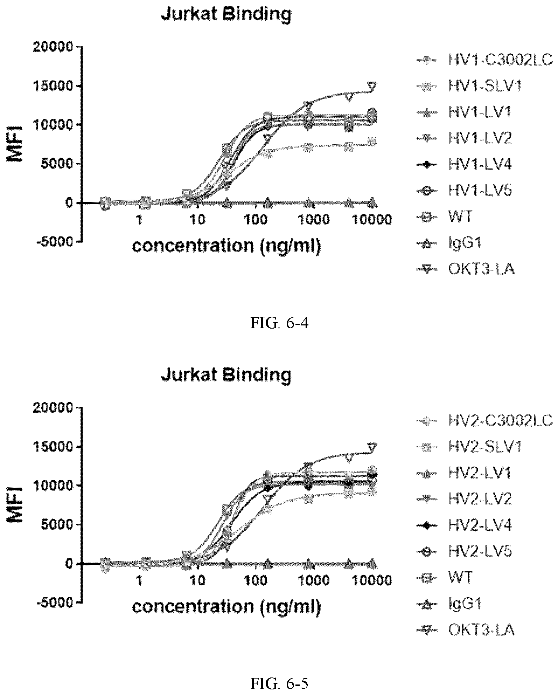

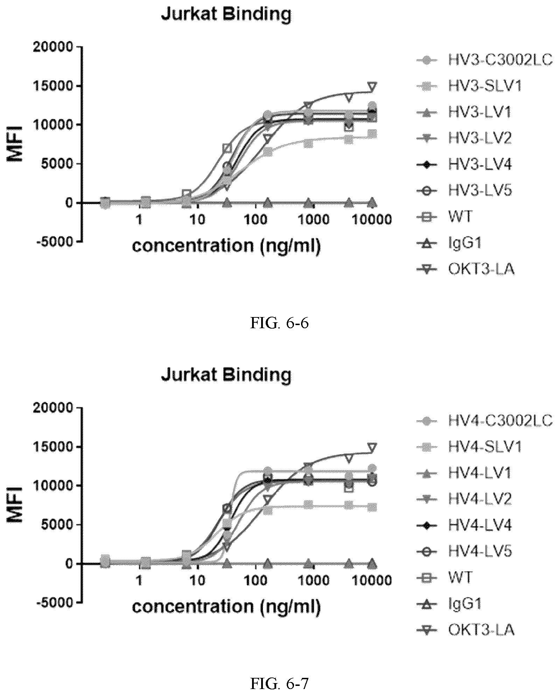

The Jurkat cells were adjusted to a concentration of 4x10⁶ cells/ml with PBS, and the cell suspension (50 µl/well) and different concentrations of CD3 antibodies (50 µl/well) were sequentially added to a U-shaped 96-well plate and mixed evenly to achieve final antibody concentrations of 10, 2.5, 1, 0.2, 0.04, 0.008, and 0 µg/ml. The cells were incubated in a 4°C refrigerator for 1.5 h. After the incubation was completed, the plate was centrifuged (300g, 5 min), then the supernatant was removed and the residue was washed with PBS (200 µl/well). Finally, FITC-conjugated AffiniPure Rat Anti-Mouse IgG (H+L) was added for staining, and flow cytometry was used for analysis and quantification. The results are shown in FIG. 6.

## Claims

1. An isolated antigen-binding protein comprising CD3-binding moiety, wherein the CD3-binding moiety comprises an amino acid sequence having at least 95% identity to HCDR1, HCDR2 and HCDR3 of heavy chain variable region, wherein the HCDR1 has an amino acid sequence set forth in any one of SEQ ID NOs: 1 and 2, the HCDR2 has an amino acid sequence set forth in any one of SEQ ID NOs: 3, 4 and 5, and the HCDR3 has an amino acid sequence set forth in SEQ ID NO: 6; and/or
the CD3-binding moiety comprises an amino acid sequence having at least 95% identity to LCDR1, LCDR2 and LCDR3 of light chain variable region, wherein the LCDR1 has an amino acid sequence set forth in any one of SEQ ID NOs: 7, 8 and 9, the LCDR2 has an amino acid sequence set forth in any one of SEQ ID NOs: 11 and 12, and the LCDR3 has an amino acid sequence set forth in SEQ ID NO: 13; and
wherein the CD3-binding moiety is capable of inducing activation of T cells.

2. The antigen-binding protein according to claim 1, comprising the CD3-binding moiety, wherein the CD3-binding moiety comprises HCDR1, HCDR2 and HCDR3 of heavy chain variable region, wherein the HCDR1 has an amino acid sequence set forth in any one of SEQ ID NOs: 1 and 2, the HCDR2 has an amino acid sequence set forth in any one of SEQ ID NOs: 3, 4 and 5, and the HCDR3 has an amino acid sequence set forth in SEQ ID NO: 6; and/or
the CD3-binding moiety comprises LCDR1, LCDR2 and LCDR3 of light chain variable region, wherein the LCDR1 has an amino acid sequence set forth in any one of SEQ ID NOs: 7, 8 and 9, the LCDR2 has an amino acid sequence set forth in any one of SEQ ID NOs: 11 and 12, and the LCDR3 has an amino acid sequence set forth in SEQ ID NO: 13; and
wherein the CD3-binding moiety is capable of inducing activation of T cells.

3. The antigen-binding protein according to any one of claims 1 to 2, comprising the CD3-binding moiety, wherein the CD3-binding moiety comprises HCDR1, HCDR2 and HCDR3 of heavy chain variable region,
wherein the HCDR1 has an amino acid sequence set forth in SEQ ID NO: 1, the HCDR2 has an amino acid sequence set forth in SEQ ID NO: 3, and the HCDR3 has an amino acid sequence set forth in SEQ ID NO: 6,
the HCDR1 has an amino acid sequence set forth in SEQ ID NO: 1, the HCDR2 has an amino acid sequence set forth in SEQ ID NO: 4, and the HCDR3 has an amino acid sequence set forth in SEQ ID NO: 6,
the HCDR1 has an amino acid sequence set forth in SEQ ID NO: 1, the HCDR2 has an amino acid sequence set forth in SEQ ID NO: 5, and the HCDR3 has an amino acid sequence set forth in SEQ ID NO: 6,
the HCDR1 has an amino acid sequence set forth in SEQ ID NO: 2, the HCDR2 has an amino acid sequence set forth in SEQ ID NO: 3, and the HCDR3 has an amino acid sequence set forth in SEQ ID NO: 6,
the HCDR1 has an amino acid sequence set forth in SEQ ID NO: 2, the HCDR2 has an amino acid sequence set forth in SEQ ID NO: 4, and the HCDR3 has an amino acid sequence set forth in SEQ ID NO: 6, or
the HCDR1 has an amino acid sequence set forth in SEQ ID NO: 2, the HCDR2 has an amino acid sequence set forth in SEQ ID NO: 5, and the HCDR3 has an amino acid sequence set forth in SEQ ID NO: 6.

4. The antigen-binding protein according to any one of claims 1 to 3, wherein the CD3-binding moiety comprises LCDR1, LCDR2 and LCDR3 of light chain variable region,
wherein the LCDR1 has an amino acid sequence set forth in SEQ ID NO: 7, the LCDR2 has an amino acid sequence set forth in SEQ ID NO: 11, and the LCDR3 has an amino acid sequence set forth in SEQ ID NO: 13,
the LCDR1 has an amino acid sequence set forth in SEQ ID NO: 8, the LCDR2 has an amino acid sequence set forth in SEQ ID NO: 11, and the LCDR3 has an amino acid sequence set forth in SEQ ID NO: 13,
the LCDR1 has an amino acid sequence set forth in SEQ ID NO: 9, the LCDR2 has an amino acid sequence set forth in SEQ ID NO: 11, and the LCDR3 has an amino acid sequence set forth in SEQ ID NO: 13,
the LCDR1 has an amino acid sequence set forth in SEQ ID NO: 7, the LCDR2 has an amino acid sequence set forth in SEQ ID NO: 12, and the LCDR3 has an amino acid sequence set forth in SEQ ID NO: 13,
the LCDR1 has an amino acid sequence set forth in SEQ ID NO: 8, the LCDR2 has an amino acid sequence set forth in SEQ ID NO: 12, and the LCDR3 has an amino acid sequence set forth in SEQ ID NO: 13, or
the LCDR1 has an amino acid sequence set forth in SEQ ID NO: 9, the LCDR2 has an amino acid sequence set forth in SEQ ID NO: 12, and the LCDR3 has an amino acid sequence set forth in SEQ ID NO: 13.

5. The antigen-binding protein according to any one of claims 1 to 4, wherein the CD3-binding moiety comprises an amino acid sequence having at least 80% identity to a heavy chain variable region having an amino acid sequence set forth in any one of SEQ ID NOs: 14 to 18; and/or the CD3-binding moiety comprises an amino acid sequence having at least 80% identity to a light chain variable region having an amino acid sequence set forth in any one of SEQ ID NOs: 19 to 23.

6. The antigen-binding protein according to any one of claims 1 to 5, wherein the CD3-binding moiety comprises a heavy chain variable region having an amino acid sequence set forth in any one of SEQ ID NOs: 14 to 18; and/or the CD3-binding moiety comprises a light chain variable region having an amino acid sequence set forth in any one of SEQ ID NOs: 19 to 23.

7. The antigen-binding protein according to any one of claims 1 to 6, wherein the CD3-binding moiety comprises:
a heavy chain variable region having an amino acid sequence set forth in SEQ ID NO: 15 and a light chain variable region having an amino acid sequence set forth in SEQ ID NO: 19,
a heavy chain variable region having an amino acid sequence set forth in SEQ ID NO: 16 and a light chain variable region having an amino acid sequence set forth in SEQ ID NO: 19,
a heavy chain variable region having an amino acid sequence set forth in SEQ ID NO: 17 and a light chain variable region having an amino acid sequence set forth in SEQ ID NO: 19,
a heavy chain variable region having an amino acid sequence set forth in SEQ ID NO: 18 and a light chain variable region having an amino acid sequence set forth in SEQ ID NO: 19,
a heavy chain variable region having an amino acid sequence set forth in SEQ ID NO: 14 and a light chain variable region having an amino acid sequence set forth in SEQ ID NO: 20,
a heavy chain variable region having an amino acid sequence set forth in SEQ ID NO: 15 and a light chain variable region having an amino acid sequence set forth in SEQ ID NO: 20,
a heavy chain variable region having an amino acid sequence set forth in SEQ ID NO: 16 and a light chain variable region having an amino acid sequence set forth in SEQ ID NO: 20,
a heavy chain variable region having an amino acid sequence set forth in SEQ ID NO: 17 and a light chain variable region having an amino acid sequence set forth in SEQ ID NO: 20,
a heavy chain variable region having an amino acid sequence set forth in SEQ ID NO: 18 and a light chain variable region having an amino acid sequence set forth in SEQ ID NO: 20,
a heavy chain variable region having an amino acid sequence set forth in SEQ ID NO: 14 and a light chain variable region having an amino acid sequence set forth in SEQ ID NO: 21,
a heavy chain variable region having an amino acid sequence set forth in SEQ ID NO: 15 and a light chain variable region having an amino acid sequence set forth in SEQ ID NO: 21,
a heavy chain variable region having an amino acid sequence set forth in SEQ ID NO: 16 and a light chain variable region having an amino acid sequence set forth in SEQ ID NO: 21,
a heavy chain variable region having an amino acid sequence set forth in SEQ ID NO: 17 and a light chain variable region having an amino acid sequence set forth in SEQ ID NO: 21,
a heavy chain variable region having an amino acid sequence set forth in SEQ ID NO: 18 and a light chain variable region having an amino acid sequence set forth in SEQ ID NO: 21,
a heavy chain variable region having an amino acid sequence set forth in SEQ ID NO: 14 and a light chain variable region having an amino acid sequence set forth in SEQ ID NO: 22,
a heavy chain variable region having an amino acid sequence set forth in SEQ ID NO: 15 and a light chain variable region having an amino acid sequence set forth in SEQ ID NO: 22,
a heavy chain variable region having an amino acid sequence set forth in SEQ ID NO: 16 and a light chain variable region having an amino acid sequence set forth in SEQ ID NO: 22,
a heavy chain variable region having an amino acid sequence set forth in SEQ ID NO: 17 and a light chain variable region having an amino acid sequence set forth in SEQ ID NO: 22,
a heavy chain variable region having an amino acid sequence set forth in SEQ ID NO: 18 and a light chain variable region having an amino acid sequence set forth in SEQ ID NO: 22,
a heavy chain variable region having an amino acid sequence set forth in SEQ ID NO: 14 and a light chain variable region having an amino acid sequence set forth in SEQ ID NO: 23,
a heavy chain variable region having an amino acid sequence set forth in SEQ ID NO: 15 and a light chain variable region having an amino acid sequence set forth in SEQ ID NO: 23,
a heavy chain variable region having an amino acid sequence set forth in SEQ ID NO: 16 and a light chain variable region having an amino acid sequence set forth in SEQ ID NO: 23,
a heavy chain variable region having an amino acid sequence set forth in SEQ ID NO: 17 and a light chain variable region having an amino acid sequence set forth in SEQ ID NO: 23,
a heavy chain variable region having an amino acid sequence set forth in SEQ ID NO: 18 and a light chain variable region having an amino acid sequence set forth in SEQ ID NO: 23,
a heavy chain variable region having an amino acid sequence set forth in SEQ ID NO: 37 and a light chain variable region having an amino acid sequence set forth in SEQ ID NO: 40,
a heavy chain variable region having an amino acid sequence set forth in SEQ ID NO: 37 and a light chain variable region having an amino acid sequence set forth in SEQ ID NO: 39,
a heavy chain variable region having an amino acid sequence set forth in SEQ ID NO: 37 and a light chain variable region having an amino acid sequence set forth in SEQ ID NO: 20,
a heavy chain variable region having an amino acid sequence set forth in SEQ ID NO: 37 and a light chain variable region having an amino acid sequence set forth in SEQ ID NO: 21,
a heavy chain variable region having an amino acid sequence set forth in SEQ ID NO: 37 and a light chain variable region having an amino acid sequence set forth in SEQ ID NO: 22,
a heavy chain variable region having an amino acid sequence set forth in SEQ ID NO: 37 and a light chain variable region having an amino acid sequence set forth in SEQ ID NO: 23,
a heavy chain variable region having an amino acid sequence set forth in SEQ ID NO: 38 and a light chain variable region having an amino acid sequence set forth in SEQ ID NO: 40,
a heavy chain variable region having an amino acid sequence set forth in SEQ ID NO: 38 and a light chain variable region having an amino acid sequence set forth in SEQ ID NO: 39,
a heavy chain variable region having an amino acid sequence set forth in SEQ ID NO: 38 and a light chain variable region having an amino acid sequence set forth in SEQ ID NO: 20,
a heavy chain variable region having an amino acid sequence set forth in SEQ ID NO: 38 and a light chain variable region having an amino acid sequence set forth in SEQ ID NO: 21,
a heavy chain variable region having an amino acid sequence set forth in SEQ ID NO: 38 and a light chain variable region having an amino acid sequence set forth in SEQ ID NO: 22,
a heavy chain variable region having an amino acid sequence set forth in SEQ ID NO: 38 and a light chain variable region having an amino acid sequence set forth in SEQ ID NO: 23,
a heavy chain variable region having an amino acid sequence set forth in SEQ ID NO: 36 and a light chain variable region having an amino acid sequence set forth in SEQ ID NO: 40,
a heavy chain variable region having an amino acid sequence set forth in SEQ ID NO: 36 and a light chain variable region having an amino acid sequence set forth in SEQ ID NO: 39,
a heavy chain variable region having an amino acid sequence set forth in SEQ ID NO: 36 and a light chain variable region having an amino acid sequence set forth in SEQ ID NO: 20,
a heavy chain variable region having an amino acid sequence set forth in SEQ ID NO: 36 and a light chain variable region having an amino acid sequence set forth in SEQ ID NO: 21,
a heavy chain variable region having an amino acid sequence set forth in SEQ ID NO: 36 and a light chain variable region having an amino acid sequence set forth in SEQ ID NO: 22,
a heavy chain variable region having an amino acid sequence set forth in SEQ ID NO: 36 and a light chain variable region having an amino acid sequence set forth in SEQ ID NO: 23,
a heavy chain variable region having an amino acid sequence set forth in SEQ ID NO: 15 and a light chain variable region having an amino acid sequence set forth in SEQ ID NO: 40,
a heavy chain variable region having an amino acid sequence set forth in SEQ ID NO: 15 and a light chain variable region having an amino acid sequence set forth in SEQ ID NO: 39,
a heavy chain variable region having an amino acid sequence set forth in SEQ ID NO: 16 and a light chain variable region having an amino acid sequence set forth in SEQ ID NO: 40,
a heavy chain variable region having an amino acid sequence set forth in SEQ ID NO: 16 and a light chain variable region having an amino acid sequence set forth in SEQ ID NO: 39,
a heavy chain variable region having an amino acid sequence set forth in SEQ ID NO: 17 and a light chain variable region having an amino acid sequence set forth in SEQ ID NO: 40,
a heavy chain variable region having an amino acid sequence set forth in SEQ ID NO: 17 and a light chain variable region having an amino acid sequence set forth in SEQ ID NO: 39,
a heavy chain variable region having an amino acid sequence set forth in SEQ ID NO: 18 and a light chain variable region having an amino acid sequence set forth in SEQ ID NO: 40, or
a heavy chain variable region having an amino acid sequence set forth in SEQ ID NO: 18 and a light chain variable region having an amino acid sequence set forth in SEQ ID NO: 39.

8. The antigen-binding protein according to any one of claims 1 to 7, wherein the antigen-binding protein is an antibody or an antigen-binding fragment thereof.

9. The antigen-binding protein according to claim 8, wherein the antibody is a monoclonal antibody.

10. The antigen-binding protein according to any one of claims 8 to 9, wherein the antibody is a chimeric antibody, a humanized antibody or a fully human antibody.

11. The antigen-binding protein according to any one of claims 8 to 10, wherein the antigen-binding fragment includes Fab, Fab', Fv fragment, F(ab')2, scFv, di-scFv and/or dAb.

12. The antigen-binding protein according to any one of claims 1 to 11, wherein the antigen-binding protein comprises a constant region from IgA, IgG, IgD, IgE or IgM.

13. The antigen-binding protein according to any one of claims 1 to 12, wherein the CD3-binding moiety comprises a heavy chain constant region of an antibody, and the heavy chain constant region of an antibody includes a constant region from human IgG.

14. The antigen-binding protein according to any one of claims 1 to 13, wherein the CD3-binding moiety comprises a heavy chain constant region of an antibody, and the heavy chain constant region of an antibody includes a heavy chain constant region from human IgG1, IgG2, IgG3 or IgG4.

15. The antigen-binding protein according to claim 14, wherein the heavy chain constant region of an antibody comprises an amino acid sequence set forth in SEQ ID NO: 24.

16. The antigen-binding protein according to any one of claims 1 to 15, wherein the CD3-binding moiety comprises a light chain constant region of an antibody, and the light chain constant region of an antibody includes a constant region of human Ig κ light chain or a constant region of human Ig λ light chain.

17. The antigen-binding protein according to claim 16, wherein the light chain constant region of an antibody comprises an amino acid sequence set forth in SEQ ID NO: 25.

18. The antigen-binding protein according to any one of claims 1 to 17, wherein the CD3-binding moiety comprises a heavy chain of an antibody having an amino acid sequence set forth in any one of SEQ ID NOs: 26 to 30 and a light chain of an antibody having an amino acid sequence set forth in any one of SEQ ID NOs: 31 to 35.

19. The antigen-binding protein according to any one of claims 1 to 18, wherein the antigen-binding protein includes a multi-specific antibody.

20. The antigen-binding protein according to any one of claims 1 to 19, wherein the multi-specific antigen-binding protein is in a form selected from the group consisting of bispecific antibody, bispecific diabody, bispecific scFv, TandAb, trivalent binding molecule and tetravalent binding molecule.

21. The antigen-binding protein according to any one of claims 19 to 20, wherein the multi-specific antibody comprises a tumor-associated antigen (TAA)-binding moiety capable of binding to at least one TAA.

22. The antigen-binding protein according to claim 21, wherein the TAA is selected from the group consisting of EPCAM, CCR5, CD19, HER2, HER3 neu, HER3, HER4, EGFR, PSMA, CEA, MUC1, MUC2, MUC3, MUC4, MUC5, MUC7, β-hCG, Lewis-Y, CD20, CD33, CD30, ganglioside GD3, 9-O-Acetyl-GD3, GM2, globo H, fucosyl GM1, Poly SA, GD2, RON, c-Met, CEACAM-6, PCTA-1, PSA, PAP, ALCAM (CD166), PECAM-1, CD151, MAGE-1, TROP2, IGF1R, TGFBR2, GHRHR, GHR, IL-6R, gp130, TNFR2, OSMRp, Patched-1, Frizzled, Robol, LTpR, CD26, CD27, CD44, CD80, CD81, CD86, CD100, CXCR4, SAS, BCMA, TWEAKR/Fn14, FGFR4, VEGFR1 ,VEGFR2, SSX1, SSX2, Carboanhydrase IX (MN/CAIX), CD44v6, Sonic Hedgehog (Shh), Wue-1, plasma cell antigen, (membrane-bound) IgE, melanoma chondroitin sulfate proteoglycan (MCSP), CCR8, TNF-alpha precursor, STEAP, mesothelin, A33 antigen, prostate stem cell antigen (PSCA), Ly-6; desmoglein 4, E-cadherin, neo-epitope, fetal acetylcholine receptor, CD25, CA19-9 marker, CA-125 marker, muellerian inhibitory substance (MIS) receptor type II, sialylated Tn antigen (sTn, TAG72), fibroblast activation antigen (FAP), endosialin, EGFRvIII, L6, SAS, CD63, TF-antigen, cora antigen, CD7, CD79b, CD22, Igα, Igβ, gp100, MT-MMPs, F19-antigen, CO-29 and EphA2.

23. The antigen-binding protein according to any one of claims 21 to 22, wherein the TAA includes HER2, CD20, CD79b or CD47.

24. The antigen-binding protein according to any one of claims 21 to 23, wherein the TAA-binding moiety comprises a heavy chain constant region of an antibody, and the heavy chain constant region of an antibody includes a heavy chain constant region from human IgG.

25. The antigen-binding protein according to any one of claims 21 to 24, wherein the TAA-binding moiety comprises a heavy chain constant region of an antibody and a light chain constant region of an antibody, wherein the heavy chain constant region of an antibody includes a heavy chain constant region from human IgG1, IgG2, IgG3 or IgG4, and the light chain constant region of an antibody includes a constant region of human Ig κ light chain or a constant region of human Ig λ light chain.

26. An isolated nucleic acid molecule encoding the antigen-binding protein according to any one of claims 1 to 25.

27. A vector comprising the isolated nucleic acid molecule according to claim 26.

28. A host cell comprising the vector according to claim 27.

29. A method for producing the antigen-binding protein according to any one of claims 1 to 25, comprising culturing the host cell according to claim 28 under a condition suitable for expressing the antigen-binding protein.

30. The method according to claim 29, wherein the host cell is selected from the group consisting of a bacterial cell, a fungal cell, a plant cell, a mammalian cell and a virus.

31. The method according to claim 30, wherein the bacterial cell is Escherichia coli, the fungal cell is a yeast cell,
the mammalian cell is selected from the group consisting of CHO, NS0, BHK and HEK293 cell, and
the cell is a hybridoma cell,
wherein the hybridoma cell is selected from a mouse hybridoma cell, a rat hybridoma cell and a rabbit hybridoma cell.

32. A pharmaceutical composition comprising the antigen-binding protein according to any one of claims 1 to 25, the isolated nucleic acid molecule according to claim 26, the vector according to claim 27 or the host cell according to claim 28, and optionally comprising a pharmaceutically acceptable carrier.

33. Use of the antigen-binding protein according to any one of claims 1 to 25, the isolated nucleic acid molecule according to claim 26, the vector according to claim 27, the host cell according to claim 28 or the pharmaceutical composition according to claim 32 in the manufacture of a medicament for treating a cancer.

34. The use according to claim 33, wherein the cancer includes a solid tumor or a hematological neoplasm.

35. The use according to claim 34, wherein the solid tumor is selected from the group consisting of prostate cancer, colorectal cancer, gastric cancer, clear cell renal cell carcinoma, bladder cancer, lung cancer, squamous cell carcinoma, glioma, breast cancer, renal cancer, neovascularization, clear cell renal cell carcinoma (ccRCC), pancreatic cancer, kidney cancer, uroepithelial carcinoma and liver-metastatic adenocarcinoma, and the hematologic neoplasm is selected from the group consisting of acute myeloid leukemia (AML), myelodysplastic syndrome (MDS), acute lymphoblastic leukemia (ALL), diffuse large B-cell lymphoma (DLBCL), chronic myelogenous leukemia (CML) and blastic plasmacytoid dendritic cell neoplasm (BPDCN).

36. A method for detecting CD3 and/or TAA in a sample using the antigen-binding protein according to any one of claims 1 to 25, comprising contacting the antigen-binding protein with the sample and detecting or quantifying a conjugate formed by the bispecific antibody with CD3 and/or TAA.

37. A method for treating a cancer in a subject in need thereof, comprising administering a therapeutically effective amount of the antigen-binding protein according to any one of claims 1 to 25 to the subject in need thereof for a time sufficient to treat the cancer.

38. The method according to claim 37, wherein the cancer is a solid tumor or a hematological neoplasm.

39. The method according to claim 37, wherein the solid tumor is selected from the group consisting of prostate cancer, colorectal cancer, gastric cancer, clear cell renal cell carcinoma, bladder cancer, lung cancer, squamous cell carcinoma, glioma, breast cancer, renal cancer, neovascularization, clear cell renal cell carcinoma (ccRCC), pancreatic cancer, kidney cancer, uroepithelial carcinoma and liver-metastatic adenocarcinoma, and the hematologic neoplasm is selected from the group consisting of acute myeloid leukemia (AML), myelodysplastic syndrome (MDS), acute lymphoblastic leukemia (ALL), diffuse large B-cell lymphoma (DLBCL), chronic myelogenous leukemia (CML) and blastic plasmacytoid dendritic cell neoplasm (BPDCN).

40. The method according to any one of claims 37 to 39, wherein the method comprises administering the antigen-binding protein in combination with a second therapeutic agent.
